# EUROPEAN PATENT APPLICATION

(11) **EP 4 578 951 A1**
(43) Date of publication of application: **02.07.2025**
(21) Application number: 23856726.7
(22) Date of filing: 25.08.2023
(51) Int. Cl.: C12N 15/68, C12N 15/11, C12N 15/85, C12P 19/34, A61K 48/00

(54) **MULTI-FRAGMENTED POLYA POLYNUCLEOTIDE STABLY EXISTING IN HOST CELL AND USE THEREOF**

(30) Priority: 26.08.2022 CN 202211032978
(71) Applicant: Shenzhen Rhegen Biotechnology Co., Ltd., Shenzhen City, Guangdong 518057 (CN); Wuhan Rhegen Biotechnology Co., Ltd., Wuhan, Hubei 430205 (CN)
(72) Inventor: HU, Yong, Shenzhen, Guangdong 518057 (CN); XU, Pan, Shenzhen, Guangdong 518057 (CN)
(74) Representative: CH Kilger Anwaltspartnerschaft mbB
(86) International application number: PCT/CN2023/114963
(87) International publication number: WO 2024/041641

(57) **Abstract**

A multi-segmented PolyA polynucleotide stably existing in a host cell and the use thereof. Provided first is a multi-segmented PolyA polynucleotide comprising no less than two PolyA sequence segments. Adjacent PolyA sequence segments are connected via a linker, wherein each PolyA sequence segment is composed of a plurality of continuous A, and each linker is composed of 1-24 nt nucleotide residues that are not all A. The multi-segmented PolyA polynucleotide can stably exist in a host cell, such as an Escherichia coli, during the passage process, so that the mRNA stability and protein output can be improved. Compared with a poly A sequence in a traditional unit form, the multi-segmented PolyA polynucleotide shows a greatly reduced recombination rate during fermentation in Escherichia coli and thereby is more advantageous for optimization of the amplification process, and therefore can be better applied to plasmid fermentation involved in transcription template preparation in production of mRNA vaccines or medicaments.

## Description

### TECHNICAL FIELD

The present disclosure relates to a PolyA-containing polynucleotide and the use thereof, especially to a polynucleotide that has a multi-segmented PolyA sequence and can stably exist in a host cell such as Escherichia coli, and the use thereof, and the present disclosure belongs to the technical field of molecular biology.

### BACKGROUND ART

Among the various solutions to COVID-19 transmission and infections, vaccine is a highly effective measure for large-scale prevention of further expansion of COVID-19. In particular, an mRNA vaccine is rapidly emerging and has a great potential in future development in the medical field due to its advantage of a short development and production period. Currently, the production of mRNAs is mainly achieved through *in vitro* transcription, and the mRNA resulting from *in vitro* transcription needs to undergo capping and tailing to exert physiological functions *in vivo.*

There are two main ways of tailing mRNAs: enzymatic and template methods. The enzymatic method involves the tailing of *in vitro* transcripts using PolyA polymerase, which is a mechanism of tailing that occurs in natural cells, but has some limitations during the research or production. The additional tailing reaction complicates the production process, and the quantity of tailing can only be controlled within a range and cannot be accurate, and difference in the tail length of the mRNA produced is a limiting factor. The template method is to construct a certain number of PolyA sequences onto a transcription template, such that tailing can be achieved during the transcription, enabling process simplification while avoiding the problem of being unable to determine the exact number of PolyA.

In the prior art, a certain number of PolyA tails are added to a transcription template, and there are two common methods for different scales: PCR and plasmid methods. The PCR method refers to adding PolyA to a transcription template through PCR using primers, with the final transcription template being a PCR product, and this method is suitable for small-scale production of mRNAs due to the limitations of the PCR system. The plasmid method involves constructing a designed PolyA sequence directly onto a plasmid, and the plasmid comprises a T7 promoter, a 5' UTR, a gene sequence of interest, a 3' UTR and the constructed PolyA sequence. A large number of transcription templates can be produced in a short time by rapid replication of the plasmid in a host cell such as Escherichia coli, enabling the production of mRNAs to an industrial scale. However, after construction of the PolyA sequence onto the plasmid, the plasmid is prone to recombination upon replication in the host cell, resulting in a change in the PolyA sequence, thus rendering the PolyA tail sequence of the mRNA produced uncontrollable, which directly affects the efficacy of mRNA medicaments or vaccines.

To improve the stability of the PolyA sequence in host cells, currently, strains that may reduce recombination are mostly used to cultivate the plasmid or the PolyA sequence is modified, so as to stabilize the PolyA sequence during replication.

### SUMMARY OF THE INVENTION

An objective of the present disclosure is to provide a polynucleotide that has a PolyA sequence and can be stably replicated in a host cell.

Another objective of the present disclosure is to provide related applications of the polynucleotide.

The present disclosure is primarily to insert different non-PolyA sequences (linkers) of certain lengths into different positions of the PolyA sequence such that the PolyA sequence is multi-segmented, thereby obtaining a polynucleotide having a multi-segmented PolyA sequence (referred to simply as a multi-segmented PolyA polynucleotide in the present disclosure). In the present disclosure, after transforming a plasmid with a multi-segmented PolyA polynucleotide into a host cell, such as Escherichia coli, and performing scaled-up fermentation, the polynucleotide can still be maintained at a highly stable level, i.e., without recombination during the passage of the host cell, and the stability of the PolyA sequence in the host cell, such as Escherichia coli, can be improved. Stable multi-segmented PolyA polynucleotides of different effective lengths can meet the different expression needs of different genes of interest. The multi-segmented PolyA polynucleotide provided by the present disclosure can significantly reduce the difficulty of plasmid construction and can be better applied to plasmid fermentation involved in transcription template preparation in the production of mRNA vaccines or medicaments. Stable, multi-segmented PolyA polynucleotides can greatly reduce the loss of PolyA sequences during plasmid fermentation, thus ensuring the efficacy of mRNA vaccines or medicaments.

In particular, in an aspect, the present disclosure provides a multi-segmented PolyA polynucleotide comprising no less than two PolyA sequence segments, wherein adjacent PolyA sequence segments are connected via a linker, each PolyA sequence segment is composed of a plurality of continuous As, and each linker is composed of 1 to 24 nt nucleotide residues that are not all As.

According to a specific embodiment of the present disclosure, the multi-segmented PolyA polynucleotide of the present disclosure comprises two, three, four or five PolyA sequence segments.

According to a specific embodiment of the present disclosure, the multi-segmented PolyA polynucleotide of the present disclosure is selected from one or more of polynucleotides having the following structures:
PolyA sequence segment 1-linker 1-PolyA sequence segment 2;
PolyA sequence segment 1-linker 1-PolyA sequence segment 2-linker 2-PolyA sequence segment 3; or
PolyA sequence segment 1-linker 1-PolyA sequence segment 2-linker 2-PolyA sequence segment 3-linker 3-PolyA sequence segment 4.

According to a specific embodiment of the present disclosure, in the multi-segmented PolyA polynucleotide of the present disclosure, each PolyA sequence segment is independently composed of 10 to 100 continuous As.

According to a specific embodiment of the present disclosure, in the multi-segmented PolyA polynucleotide of the present disclosure, each PolyA sequence segment is independently composed of 30 to 100, for example, 30, 40, 50, 60, 70, 80, 90 or 100 continuous As.

Those skilled in the art will appreciate that, unless specifically noted, the number of base A of the PolyA sequence segment described in the present disclosure is an approximate number, particularly when the number of base A of the PolyA sequence segment is a multiple of 10, an error of about ± 5, preferably ± 3, more preferably ± 2 or ± 1 base A is allowed on the basis of the numerical value described, for example, the PolyA sequence segments is composed of 30 continuous As, meaning that PolyA sequence segments actually composed of 25, 26, 27, 28, 29, 30, 31, 32, 33 and/or 34 continuous As are included; for example, the PolyA sequence segment is composed of 70 continuous As, meaning that PolyA sequence segments actually composed of 65, 66, 67, 68, 69, 70, 71, 72, 73 and/or 74 continuous As are included; and so on.

According to a specific embodiment of the present disclosure, in the multi-segmented PolyA polynucleotide of the present disclosure, the PolyA sequence segment 1 is composed of 30 to 60 continuous As.

According to a specific embodiment of the present disclosure, in the multi-segmented PolyA polynucleotide of the present disclosure, the total A number of all the PolyA sequence segments is 30 to 300, preferably 60 to 240, more preferably 100 to 200.

According to a specific embodiment of the present disclosure, in the multi-segmented PolyA polynucleotide of the present disclosure, each linker is independently composed of 1 to 18 nt, preferably 1 to 12 nt nucleotide residues that are not all As.

According to a specific embodiment of the present disclosure, in the multi-segmented PolyA polynucleotide of the present disclosure, each linker is independently composed of G, or has a G content of 20% to 33% of the total bases in the linker sequence.

According to a specific embodiment of the present disclosure, in the multi-segmented PolyA polynucleotide of the present disclosure, each linker is independently selected from the nucleotides as shown in the following sequences:
G;
C;
GGGGGG;
ATGCAT;
GCATATGACT (SEQ ID No. 53);
CAGTAATGAC (SEQ ID No. 54);
AGTCATATGC (SEQ ID No. 55);
GTCATTACTG (SEQ ID No. 56);
TGTCAGATAC (SEQ ID No. 57);
GCTCATATGC (SEQ ID No. 58);
GCATATATGC (SEQ ID No. 59);
CGCCATTAGAGG (SEQ ID No. 60);
ATGCATGATATC (SEQ ID No. 61);
TGCAACATCGAT (SEQ ID No. 62);
CCTCTAATGGCG (SEQ ID No. 63); and
CAACCCCTGATTGTGTCCGCATCT (SEQ ID No. 64).

According to a specific embodiment of the present disclosure, in the multi-segmented PolyA polynucleotide of the present disclosure, the sum of the lengths of all linkers is 1 to 48 nt, more preferably 2 to 24 nt.

According to a specific embodiment of the present disclosure, in the multi-segmented PolyA polynucleotide of the present disclosure, the sum of the lengths of all linkers accounts for not more than 20% of the total length of the polynucleotides, preferably not more than 15%, further preferably not more than 10%, further more preferably not more than 5%.

According to a specific embodiment of the present disclosure, the multi-segmented PolyA polynucleotide of the present disclosure comprises one or more of polynucleotides of any one of sequences as shown in SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9, SEQ ID NO. 10, SEQ ID NO. 11, SEQ ID NO. 12, SEQ ID NO. 13, SEQ ID NO. 14, SEQ ID NO. 15, SEQ ID NO. 16, SEQ ID NO. 17, SEQ ID NO. 18, SEQ ID NO. 19, SEQ ID NO. 20, SEQ ID NO. 21, SEQ ID NO. 22, SEQ ID NO. 23, SEQ ID NO. 24, SEQ ID NO. 25, SEQ ID NO. 26, SEQ ID NO. 27, SEQ ID NO. 28, SEQ ID NO. 29, SEQ ID NO. 30, SEQ ID NO. 31, SEQ ID NO. 32, SEQ ID NO. 33, SEQ ID NO. 34, SEQ ID NO. 35, SEQ ID NO. 36, SEQ ID NO. 37, SEQ ID NO. 38, SEQ ID NO. 39, SEQ ID NO. 40, SEQ ID NO. 41, SEQ ID NO. 42, SEQ ID NO. 43, SEQ ID NO. 44, SEQ ID NO. 45, SEQ ID NO. 46, SEQ ID NO. 47, SEQ ID NO. 48, SEQ ID NO. 49, SEQ ID NO. 50, SEQ ID NO. 51, SEQ ID NO. 52, SEQ ID NO. 77, SEQ ID NO. 78, SEQ ID NO. 79, SEQ ID NO. 80, and SEQ ID NO. 81.

In another aspect, the present disclosure also provides a vector comprising the multi-segmented PolyA polynucleotide described in the present disclosure. The multi-segmented PolyA polynucleotide described in the present disclosure can be inserted into a plasmid vector using any feasible method in the art, thereby constructing a vector of the present disclosure inserted with the multi-segmented PolyA polynucleotide. For specific construction, enzymatic cleavage sites can be added to the ends of the multi-segmented PolyA polynucleotide as required.

In another aspect, the present disclosure also provides a host cell comprising the multi-segmented PolyA polynucleotide described in the present disclosure or the vector described in the present disclosure (i.e., the vector inserted with the multi-segmented PolyA polynucleotide).

In another aspect, the present disclosure also provides the use of the multi-segmented PolyA polynucleotide, the vector, and the host cell in the production of an mRNA of interest through *in vitro* transcription. The mRNA of interest has a poly-A tail formed by the transcription of the multi-segmented PolyA polynucleotide described in the present disclosure.

Therefore, the present disclosure also provides an mRNA comprising a poly-A tail formed by *in vitro* transcription of the multi-segmented PolyA polynucleotide described in the present disclosure. The poly-A tail is the mRNA sequence corresponding to the multi-segmented PolyA polynucleotide described in the present disclosure.

According to a specific embodiment of the present disclosure, the mRNA of interest is used for the production of mRNA vaccines or medicaments. The multi-segmented PolyA polynucleotide provided by the present disclosure can be better applied to plasmid fermentation involved in transcription template preparation in the production of mRNA vaccines or medicaments. Stable, multi-segmented PolyA polynucleotides can greatly reduce the loss of PolyA sequences during plasmid fermentation, thus ensuring the efficacy of mRNA vaccines or medicaments.

In another aspect, the present disclosure also provides a method for identifying the stability of a multi-segmented PolyA polynucleotide in a host cell, the method comprising:
1) cloning the multi-segmented PolyA polynucleotide described in the present disclosure into a universal plasmid vector to obtain a plasmid vector inserted with the multi-segmented PolyA polynucleotide;
2) transforming the plasmid vector inserted with the multi-segmented PolyA polynucleotide into a host cell such as Escherichia coli to obtain a recombinant cell, spreading the recombinant cell on a solid medium in a plate to obtain single colonies, picking the single colonies for preliminary identification by PCR, and screening for stable colonies without recombination; wherein, preferably, PCR products are identified by using 2% to 3% agarose gel electrophoresis; and
3) performing scaled-up fermentation culture of the stable bacteria without recombination screened out by the preliminary identification, spreading a serial-diluted culture broth on a solid medium in a plate, and carrying out colony PCR to identify the passage stability.

In some specific embodiments of the present disclosure, the method for identifying the stability of the multi-segmented PolyA polynucleotide of the present disclosure in the host cell comprises:
1) preparing a gene segment with the above sequence by gene synthesis, constructing the gene segment onto pUC-GW-kana universal plasmid and synthesizing the corresponding universal primers;
2) performing gradient annealing PCR using the plasmid as a template to identify an optimal annealing temperature;
3) transforming the plasmid into Escherichia coli (including but not limited to DH5α, stbl2, stbl3, stable) by conventional transformation for Escherichia coli, spreading Escherichia coli after transformation respectively on different LB plates containing kana (kanamycin) (at a final concentration of 50 µg/ml, hereinafter the kana contained being at this concentration, which will not be elaborated further), and incubating the plates at 30 to 37°C for 12 to 20 h;
4) picking single colonies from each plate to 20 µl of a kana-containing liquid medium, and thoroughly mixing them to prepare a colony suspension;
5) formulating a colony PCR reaction system for identifying the stability of the sequence, wherein the colony PCR system is 20 µl and comprises the following components: 7 µl of RNA-free water; 0.5 µl of each of primer 1 and primer 2; 10 µl of Enzyme Mix; and 2 µl of the colony suspension;
6) identifying the PCR products obtained in step 5) by 2% to 3% agarose gel electrophoresis; and determining the stability of the corresponding PolyA sequence in Escherichia coli by analyzing the electrophoresis results;
7) when the recombination rate is less than 15%, selecting the bacterial suspension without recombination and aspirating 5 µl of the bacterial suspension to inoculate into 100 ml of a kana-containing medium, placing the medium in a shaker at 220 rpm at 30°C for scaled-up culture for 12 to 16 h, then performing 10⁴-fold dilution and spreading the bacterial suspension onto a kana-containing solid medium in a plate, invertedly incubating the plate in an incubator at 30°C for 20 h, and repeating operations of step 4) to step 6) to further verify the stability of the PolyA sequence during scaled-up culture; and
8) inoculating the monoclonal colonies on the plate obtained in the further validation to 3 ml of a Kana-containing medium and culturing in a shaker at 30 to 37°C for 12 to 20 h, then carrying out the plasmid miniprep, entrusting the plasmid extracted by miniprep to an authoritative biological company for sequencing, and performing sequence alignment analysis of sequencing results, which can further serve as another aspect of evidence for sequence stability.

Specific experiments of the present disclosure show that the multi-segmented PolyA polynucleotide provided by the present disclosure can significantly reduce phenomena of alteration or loss of PolyA sequences due to homologous recombination during the passage of Escherichia coli, thereby greatly improving the quality of mRNA produced. PolyA sequences of different effective lengths can meet expression needs of different genes of interest, and the stable passage of such sequences in Escherichia coli also provides the possibility for plasmid scaled-up production and fermentation, which brings great convenience for the simplification of the production process and the increase of yield. The multi-segmented PolyA polynucleotide provided by the present disclosure can be better applied to plasmid fermentation involved in transcription template preparation in the production of mRNA vaccines or medicaments.

### BRIEF DESCRIPTION OF THE DRAWINGS

FIG. 1 is a schematic diagram of the designed structure of the multi-segmented PolyA polynucleotide of the present disclosure.
FIG. 2 is the plasmid map of pUC-GW-kana.
FIG. 3 shows the results for exploring the optimal annealing temperature of primers cgacggccagtgaattgac (SEQ ID No. 65) and cagctatgaccatgctcgag (SEQ ID No. 66) by using the universal plasmid pUC-GW-kana with the sequence as shown in SEQ ID No. 1 as a template.
FIG. 4 shows comparative results of the stability of some of the multi-segmented PolyA polynucleotides of the present disclosure in Escherichia coli stbl3.
FIG. 5 shows comparative results of the stability of some of the multi-segmented PolyA polynucleotides of the present disclosure in Escherichia coli stbl2.
FIG. 6 shows the results of sequencing and alignment of some of the multi-segmented PolyA polynucleotides of the present disclosure, after the plasmid miniprep of the strains of Escherichia coli stbl2 which have undergone the scaled-up fermentation.
FIG. 7 shows the results of correlation analysis between each of sequence features (which are disassembled into linker amount, position, length and the contents of G, C, and T) and the stability of the multi-segmented PolyA polynucleotides, based on the data described in FIGS. 4 and 5.
FIG. 8 shows the results of fluorescence microscopy of 293T/17 cells transfected with a corresponding mRNA, wherein the mRNA is obtained by adding some of the multi-segmented PolyA polynucleotides of the present disclosure to a DNA template encoding and expressing GFP using the plasmid method or PCR method and performing IVT.
FIG. 9 shows the results of flow cytometry detection of the cell sample described in FIG. 8 after processing (specific detection data is shown in the table in the figure).
FIG. 10 shows the detection results of the expression level of mRNAs corresponding to the multi-segmented PolyAs as shown in SEQ ID No. 77 to SEQ ID No. 81.
FIG. 11 shows the stability detection data for A30G70 and A30L70 after extreme scaled-up culture.

### DETAILED DESCRIPTION OF THE INVENTION

In order to understand the technical features, objectives and beneficial effects of the present disclosure more clearly, the technical solutions of the present disclosure are described in detail as below, but cannot be construed as limitations on the implementable scope of the present disclosure. The reagent raw materials used in each experiments below are commercially available, and experimental methods without specifying specific conditions are conventional methods and conditions well known in the art, or are conducted according to conditions suggested by an instrument manufacturer.

Unless specifically defined otherwise, all the technical and scientific terms used herein have the same meanings as commonly understood by those of ordinary skill in the relevant art.

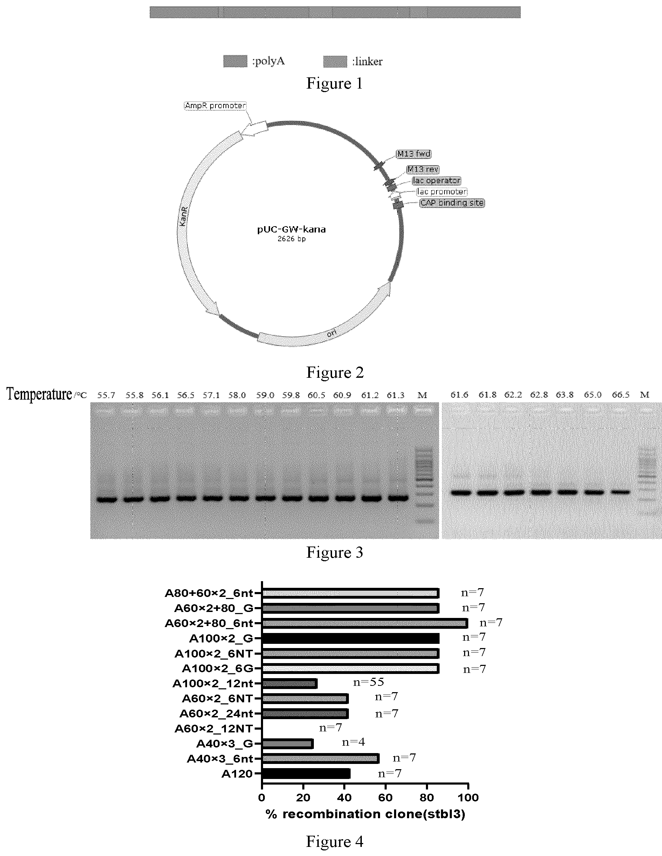

The present disclosure provides a multi-segmented PolyA polynucleotide, which has a structural design as shown in FIG. 1. In particular, the present disclosure provides multi-segmented PolyA polynucleotides as shown in SEQ ID No. 1 to SEQ ID No. 52, and SEQ ID No. 77 to SEQ ID No. 81 in Table 1 (wherein the number of base A of PolyA sequence segments shown in SEQ ID No. 1 to SEQ ID No. 52 has an error of ± 1 base A, and the number of base A of PolyA sequence segments shown in SEQ ID No. 77 to SEQ ID No. 81 is an exact value).

**Table 1**

| SEQ ID No. | Sequence Name | Sequence |
|---|---|---|
| 1 | A120 | AAAAAAAAAAAAAAAAAAAAAAAAAAAAAAAAAAAAAAAAAAAAAAAAAAAAAAAAAAAAAAAAA AAAAAAAAAAAAAAAAAAAAAAAAAAAAAAAAAAAAAAAAAAAAAAAAAAAAAAA |
| 2 | A40×3_6nt | AAAAAAAAAAAAAAAAAAAAAAAAAAAAAAAAAAAAAAAAATGCATAAAAAAAAAAAAAAAAAAA AAAAAAAAAAAAAAAAAAAAAAGATATCAAAAAAAAAAAAAAAAAAAAAAAAAAAAAAAAAAAAA AA |
| 3 | A40×3_G | AAAAAAAAAAAAAAAAAAAAAAAAAAAAAAAAAAAAAAAAAAGAAAAAAAAAAAAAAAAAAAAAAAA AAAAAAAAAAAAAAAAGAAAAAAAAAAAAAAAAAAAAAAAAAAAAAAAAAAAAAAAAAA |
| 4 | A60×2_6nt | AAAAAAAAAAAAAAAAAAAAAAAAAAAAAAAAAAAAAAAAAAAAAAAAAAAAAAAAAAAAAAAAAATGCA TAAAAAAAAAAAAAAAAAAAAAAAAAAAAAAAAAAAAAAAAAAAAAAAAAAAAAAAAAAAAAAAA |
| 5 | A60×2_12nt | AAAAAAAAAAAAAAAAAAAAAAAAAAAAAAAAAAAAAAAAAAAAAAAAAAAAAAACGCCA TTAGAGGAAAAAAAAAAAAAAAAAAAAAAAAAAAAAAAAAAAAAAAAAAAAAAAAAAAAA AA |
| 6 | A60×2_24nt | AAAAAAAAAAAAAAAAAAAAAAAAAAAAAAAAAAAAAAAAAAAAAAAAAAAAAAAAAAAACAACC CCTGATTGTGTCCGCATCTAAAAAAAAAAAAAAAAAAAAAAAAAAAAAAAAAAAAAAAAAAAAAA AAAAAAAAAAAAAA |
| 7 | A60×2_12nt(1) | AAAAAAAAAAAAAAAAAAAAAAAAAAAAAAAAAAAAAAAAAAAAAAAAAAAAAAAAAAAAAAACCTCT AATGGCGAAAAAAAAAAAAAAAAAAAAAAAAAAAAAAAAAAAAAAAAAAAAAAAAAAAAAAAAAAAAA AA |
| 8 | A60×2_12nt(2) | AAAAAAAAAAAAAAAAAAAAAAAAAAAAAAAAAAAAAAAAAAAAAAAAAAAAAAAAAAAAATGCA TGATATCAAAAAAAAAAAAAAAAAAAAAAAAAAAAAAAAAAAAAAAAAAAAAAAAAAAAAAAAAA AA |
| 9 | A60×2_12nt(3) | AAAAAAAAAAAAAAAAAAAAAAAAAAAAAAAAAAAAAAAAAAAAAAAAAAAAAAATGCAA CATCGATAAAAAAAAAAAAAAAAAAAAAAAAAAAAAAAAAAAAAAAAAAAAAAAAAAAA AA |
| 10 | A60×2_G | AAAAAAAAAAAAAAAAAAAAAAAAAAAAAAAAAAAAAAAAAAAAAAAAAAAAAAAAAAAAAAAAGAAAA AAAAAAAAAAAAAAAAAAAAAAAAAAAAAAAAAAAAAAAAAAAAAAAAAAAAAAAAAAAA |
| 11 | A60+80+60_G | AAAAAAAAAAAAAAAAAAAAAAAAAAAAAAAAAAAAAAAAAAAAAAAAAAAAAAAAAAAAGAAAA AAAAAAAAAAAAAAAAAAAAAAAAAAAAAAAAAAAAAAAAAAAAAAAAAAAAAAAAAAAAAAAAA AAAAAAAAAAAGAAAAAAAAAAAAAAAAAAAAAAAAAAAAAAAAAAAAAAAAAAAAAAAAAAAAA AAAAAAA |
| 12 | A60×2+80_G | AAAAAAAAAAAAAAAAAAAAAAAAAAAAAAAAAAAAAAAAAAAAAAAAAAAAAAAAAAAAGAAAA AAAAAAAAAAAAAAAAAAAAAAAAAAAAAAAAAAAAAAAAAAAAAAAAAAAAAAAAGAAAAAAAA AAAAAAAAAAAAAAAAAAAAAAAAAAAAAAAAAAAAAAAAAAAAAAAAAAAAAAAAAAAAAAAAA AAAAAAA |
| 13 | A100×2_6G | AAAAAAAAAAAAAAAAAAAAAAAAAAAAAAAAAAAAAAAAAAAAAAAAAAAAAAAAAAAAAAAA AAAAAAAAAAAAAAAAAAAAAAAAAAAAAAAAAAAGGGGGGAAAAAAAAAAAAAAAAAAAAAAAA AAAAAAAAAAAAAAAAAAAAAAAAAAAAAAAAAAAAAAAAAAAAAAAAAAAAAAAAAAAAAAAA AAAAAAAAAAA |
| 14 | A100×2_6nt | AAAAAAAAAAAAAAAAAAAAAAAAAAAAAAAAAAAAAAAAAAAAAAAAAAAAAAAAAAAAAAAAA AAAAAAAAAAAAAAAAAAAAAAAAAAAAAAAAAAATGCATAAAAAAAAAAAAAAAAAAAAAAAA AAAAAAAAAAAAAAAAAAAAAAAAAAAAAAAAAAAAAAAAAAAAAAAAAAAAAAAAAAAAAAAAA AAAAAAAAAAA |
| 15 | A100×2_12nt | AAAAAAAAAAAAAAAAAAAAAAAAAAAAAAAAAAAAAAAAAAAAAAAAAAAAAAAAAAAAAAAAA AAAAAAAAAAAAAAAAAAAAAAAAAAAAAAAAAAACGCCATTAGAGGAAAAAAAAAAAAAAAAAA AAAAAAAAAAAAAAAAAAAAAAAAAAAAAAAAAAAAAAAAAAAAAAAAAAAAAAAAAAAAAAAAA AAAAAAAAAAAAAAAAA |
| 16 | A100×2_24nt | AAAAAAAAAAAAAAAAAAAAAAAAAAAAAAAAAAAAAAAAAAAAAAAAAAAAAA AAAAAAAAAAAAAAAAAAAAAAAAAAAAACAACCCCTGATTGTGTCCGCATCTAAAAAA AAAAAAAAAAAAAAAAAAAAAAAAAAAAAAAAAAAAAAAAAAAAAAAAAAAAAA AAAAAAAAAAAAAAAAAAAAAAAA |
| 17 | A100×2_G | AAAAAAAAAAAAAAAAAAAAAAAAAAAAAAAAAAAAAAAAAAAAAAAAAAAAAAAAAAAAAAAAAA AAAAAAAAAAAAAAAAAAAAAAAAAAAAAAAAAAAAGAAAAAAAAAAAAAAAAAAAAAAAAAAAAA AAAAAAAAAAAAAAAAAAAAAAAAAAAAAAAAAAAAAAAAAAAAAAAAAAAAAAAAAAAAAAAAAA AAAAAA |
| 18 | A60+80+60_6nt | AAAAAAAAAAAAAAAAAAAAAAAAAAAAAAAAAAAAAAAAAAAAAAAAAAAAAAAAAAAAATGCA TAAAAAAAAAAAAAAAAAAAAAAAAAAAAAAAAAAAAAAAAAAAAAAAAAAAAAAAAAAAAAAAA AAAAAAAAAAAAAAAAATGCATAAAAAAAAAAAAAAAAAAAAAAAAAAAAAAAAAAAAAAAAAAA AAAAAAAAAAAAAAAAA |
| 19 | A80+60×2_6nt | AAAAAAAAAAAAAAAAAAAAAAAAAAAAAAAAAAAAAAAAAAAAAAAAAAAAAAAAAAAAAAAAAAAAAA AAAAAAAAAAAAAAAAATGCATAAAAAAAAAAAAAAAAAAAAAAAAAAAAAAAAAAAAAAAAAAAAAAAA AAAAAAAAAAAAAAAAATGCATAAAAAAAAAAAAAAAAAAAAAAAAAAAAAAAAAAAAAAAAAAAAAAAA |
| | | AAAAAAAAAAAAAAAAAA |
| 20 | A60×2+80_6nt | AAAAAAAAAAAAAAAAAAAAAAAAAAAAAAAAAAAAAAAAAAAAAAAAAAAAAAAAAAAAAAAAAAAATGCA TAAAAAAAAAAAAAAAAAAAAAAAAAAAAAAAAAAAAAAAAAAAAAAAAAAAAAAAAAAAAAAAAAAAATGC ATAAAAAAAAAAAAAAAAAAAAAAAAAAAAAAAAAAAAAAAAAAAAAAAAAAAAAAAAAAAAAAAAAAAAAA AAAAAAAAAAAAAAAAAAA |
| 21 | A 80×2-12nt | AAAAAAAAAAAAAAAAAAAAAAAAAAAAAAAAAAAAAAAAAAAAAAAAAAAAAAAAAAAAAAAAAAAAAA AAAAAAAAAAAAAAAACGCCATTAGAGGAAAAAAAAAAAAAAAAAAAAAAAAAAAAAAAAAAAAAAAAAA AAAAAAAAAAAAAAAAAAAAAAAAAAAAAAAAAAAAAAAAAAAAA |
| 22 | A 80×2-10nt | AAAAAAAAAAAAAAAAAAAAAAAAAAAAAAAAAAAAAAAAAAAAAAAAAAAAAAAAAAAAAAAAAAAA AAAAAAAAAAAAAAAGCATATGACTAAAAAAAAAAAAAAAAAAAAAAAAAAAAAAAAAAAAAAAAAAA AAAAAAAAAAAAAAAAAAAAAAAAAAAAAAAAAAAAAAAAAA |
| 23 | A30+100+30-10nt | AAAAAAAAAAAAAAAAAAAAAAAAAAAAAAAGCATATGACTAAAAAAAAAAAAAAAAAAAAAAAAAAA AAAAAAAAAAAAAAAAAAAAAAAAAAAAAAAAAAAAAAAAAAAAAAAAAAAAAAAAAAAAAAAAAAAA AAAAAAAAAAGCATATGACTAAAAAAAAAAAAAAAAAAAAAAAAAAAAAAAA |
| 24 | A30+30+100-10nt | AAAAAAAAAAAAAAAAAAAAAAAAAAAAAGCATATGACTAAAAAAAAAAAAAAAAAAAAAAAAA AAAAAGCATATGACTAAAAAAAAAAAAAAAAAAAAAAAAAAAAAAAAAAAAAAAAAAAAAAAA AAAAAAAAAAAAAAAAAAAAAAAAAAAAAAAAAAAAAAAAAAAAAAAA |
| 25 | A30+100+30-12nt | AAAAAAAAAAAAAAAAAAAAAAAAAAAACGCCATTAGAGGAAAAAAAAAAAAAAAAAAAAAAAA AAAAAAAAAAAAAAAAAAAAAAAAAAAAAAAAAAAAAAAAAAAAAAAAAAAAAAAAAAAAAAAA AAAAAAAAAAAACGCCATTAGAGGAAAAAAAAAAAAAAAAAAAAAAAAAAAAA |
| 26 | A30+30+100-12nt | AAAAAAAAAAAAAAAAAAAAAAAAAAAAACGCCATTAGAGGAAAAAAAAAAAAAAAAAAA AAAAAAACGCCATTAGAGGAAAAAAAAAAAAAAAAAAAAAAAAAAAAAAAAAAAAAAAAAA AAAAAAAAAAAAAAAAAAAAAAAAAAAAAAAAAAAAAAAAAAAAAAAAAA |
| 27 | A30+60×2-12nt | AAAAAAAAAAAAAAAAAAAAAAAAAAAACGCCATTAGAGGAAAAAAAAAAAAAAAAAAAAAA AAAAAAAAAAAAAAAAAAAAAAAAAAAAAAAAAAAACGCCATTAGAGGAAAAAAAAAAAAAA AAAAAAAAAAAAAAAAAAAAAAAAAAAAAAAAAAAAAAAAA |
| 28 | A60×2+30-12nt | AAAAAAAAAAAAAAAAAAAAAAAAAAAAAAAAAAAAAAAAAAAAAAAAAAAAAAAAAAAAAAAAACGCCA TTAGAGGAAAAAAAAAAAAAAAAAAAAAAAAAAAAAAAAAAAAAAAAAAAAAAAAAAAAAAAAAAAAAAA AACGCCATTAGAGGAAAAAAAAAAAAAAAAAAAAAAAAAAAAAAAAA |
| 29 | A30-10nt+ 60-12nt-30 | AAAAAAAAAAAAAAAAAAAAAAAAAAAAGCATATGACTAAAAAAAAAAAAAAAAAAAAAAAAAA AAAAAAAAAAAAAAAAAAAAAAAAAAAAAAAAAACGCCATTAGAGGAAAAAAAAAAAAAAAAAA AAAAAAAAAAAA |
| 30 | A80×2-12nt-1 | AAAAAAAAAAAAAAAAAAAAAAAAAAAAAAAAAAAAAAAAAAAAAAAAAAAAAAAAAAAAAAAAAAAAAA AAAAAAAAAAAAAAACCTCTAATGGCGAAAAAAAAAAAAAAAAAAAAAAAAAAAAAAAAAAAAAAAAAAA AAAAAAAAAAAAAAAAAAAAAAAAAAAAAAAAAAAAAAAAAAAAA |
| 31 | A80×2-12nt-2 | AAAAAAAAAAAAAAAAAAAAAAAAAAAAAAAAAAAAAAAAAAAAAAAAAAAAAAAAAAAAAAAAAAAA AAAAAAAAAAAAAAAATGCATGATATCAAAAAAAAAAAAAAAAAAAAAAAAAAAAAAAAAAAAAAAAA AAAAAAAAAAAAAAAAAAAAAAAAAAAAAAAAAAAAAAAAAAAA |
| 32 | A80×2-10nt-3 | AAAAAAAAAAAAAAAAAAAAAAAAAAAAAAAAAAAAAAAAAAAAAAAAAAAAAAAAAAAAAAAAAAAAAAAA AAAAAAAAAAAAAAAAACAGTAATGACAAAAAAAAAAAAAAAAAAAAAAAAAAAAAAAAAAAAAAAAAAAA AAAAAAAAAAAAAAAAAAAAAAAAAAAAAAAAAAAAAAAAAAAA |
| 33 | A80×2-G | AAAAAAAAAAAAAAAAAAAAAAAAAAAAAAAAAAAAAAAAAAAAAAAAAAAAAAAAAAAAAAAAAAAAAA AAAAAAAAAAAAAAAAGAAAAAAAAAAAAAAAAAAAAAAAAAAAAAAAAAAAAAAAAAAAAAAAAAAAA AAAAAAAAAAAAAAAAAAAAAAAAAAAAAAAAA |
| 34 | A80×2-10nt-1 | AAAAAAAAAAAAAAAAAAAAAAAAAAAAAAAAAAAAAAAAAAAAAAAAAAAAAAAAAAAAAAAAAAAAAA AAAAAAAAAAAAAAAAGTCATATGCAAAAAAAAAAAAAAAAAAAAAAAAAAAAAAAAAAAAAAAAAAAAA |
| | | AAAAAAAAAAAAAAAAAAAAAAAAAAAAAAAAAAAAAAAA |
| 35 | A80×2-10nt-2 | AAAAAAAAAAAAAAAAAAAAAAAAAAAAAAAAAAAAAAAAAAAAAAAAAAAAAAAAAAAAAAAAAAAAAA AAAAAAAAAAAAAAAAGTCATTACTGAAAAAAAAAAAAAAAAAAAAAAAAAAAAAAAAAAAAAAAAAAAA AAAAAAAAAAAAAAAAAAAAAAAAAAAAAAAAAAAAAAAAAAA |
| 36 | A80×2-12nt-3 | AAAAAAAAAAAAAAAAAAAAAAAAAAAAAAAAAAAAAAAAAAAAAAAAAAAAAAAAAAAAAAAAAAAAAAAA AAAAAAAAAAAAAAATGCAACATCGATAAAAAAAAAAAAAAAAAAAAAAAAAAAAAAAAAAAAAAAAAAAAAA AAAAAAAAAAAAAAAAAAAAAAAAAAAAAAAAAAAAAAAAAAAAAA |
| 37 | A30-10nt-2+6₀×2-12nt-2 | AAAAAAAAAAAAAAAAAAAAAAAAAAAAGTCATTACTGAAAAAAAAAAAAAAAAAAAAAAAAAAA AAAAAAAAAAAAAAAAAAAAAAAAAAAAAAAAAAAAAATGCATGATATCAAAAAAAAAAAAAAAAAA AAAAAAAAAAAAAAAAAAAAAAAAAAAAAAAAAAAAAAAAA |
| 38 | A30-12nt-100 | AAAAAAAAAAAAAAAAAAAAAAAAAAAAACGCCATTAGAGGAAAAAAAAAAAAAAAAAAAAAAA AAAAAAAAAAAAAAAAAAAAAAAAAAAAAAAAAAAAAAAAAAAAAAAAAAAAAAAAAAAAAAAA AAAAAAAAAAAA |
| 39 | A30-12nt-1-100 | AAAAAAAAAAAAAAAAAAAAAAAAAAACCTCTAATGGCGAAAAAAAAAAAAAAAAAAAAAAAAA AAAAAAAAAAAAAAAAAAAAAAAAAAAAAAAAAAAAAAAAAAAAAAAAAAAAAAAAAAAAAAAA AAAAAAAAAAAA |
| 40 | A30-10nt-100 | AAAAAAAAAAAAAAAAAAAAAAAAAAAAAAGCATATGACTAAAAAAAAAAAAAAAAAAAAAAAAA AAAAAAAAAAAAAAAAAAAAAAAAAAAAAAAAAAAAAAAAAAAAAAAAAAAAAAAAAAAAAAAAAA AAAAAAAAAA |
| 41 | A30-10nt-5-100 | AAAAAAAAAAAAAAAAAAAAAAAAAAAAAATGTCAGATACAAAAAAAAAAAAAAAAAAAAAAAAA AAAAAAAAAAAAAAAAAAAAAAAAAAAAAAAAAAAAAAAAAAAAAAAAAAAAAAAAAAAAAAAAAA AAAAAAAAAA |
| 42 | A(30-10nt)×4 | AAAAAAAAAAAAAAAAAAAAAAAAAAAAAGCATATGACTAAAAAAAAAAAAAAAAAAAAAAAAA AAAAAGCATATGACTAAAAAAAAAAAAAAAAAAAAAAAAAAAAAGCATATGACTAAAAAAAAAA AAAAAAAAAAAAAAAAAAAA |
| 43 | A(30-10nt-5)×4 | AAAAAAAAAAAAAAAAAAAAAAAAAAAAAATGTCAGATACAAAAAAAAAAAAAAAAAAAAAAAAA AAAAATGTCAGATACAAAAAAAAAAAAAAAAAAAAAAAAAAAAAATGTCAGATACAAAAAAAAAA AAAAAAAAAAAAAAAAAAAA |
| 44 | A30L70 | AAAAAAAAAAAAAAAAAAAAAAAAAAAAAGCATATGACTAAAAAAAAAAAAAAAAAAAAAAA AAAAAAAAAAAAAAAAAAAAAAAAAAAAAAAAAAAAAAAAAAA |
| 45 | A30L1-70 | AAAAAAAAAAAAAAAAAAAAAAAAAAAAAGCTCATATGCAAAAAAAAAAAAAAAAAAAAAAA AAAAAAAAAAAAAAAAAAAAAAAAAAAAAAAAAAAAAAAAAAA |
| 46 | A30L2-70 | AAAAAAAAAAAAAAAAAAAAAAAAAAAAGCATATATGCAAAAAAAAAAAAAAAAAAAAAAAA AAAAAAAAAAAAAAAAAAAAAAAAAAAAAAAAAAAAAAAAAAA |
| 47 | A30G70 | AAAAAAAAAAAAAAAAAAAAAAAAAAAAAAGAAAAAAAAAAAAAAAAAAAAAAAAAAAAAAAAA AAAAAAAAAAAAAAAAAAAAAAAAAAAAAAAAAAAA |
| 48 | A40G70 | AAAAAAAAAAAAAAAAAAAAAAAAAAAAAAAAAAAAAAGAAAAAAAAAAAAAAAAAAAAAAA AAAAAAAAAAAAAAAAAAAAAAAAAAAAAAAAAAAAAAAAAAAA |
| 49 | A30L70L70 | AAAAAAAAAAAAAAAAAAAAAAAAAAAAGCATATGACTAAAAAAAAAAAAAAAAAAAAAA AAAAAAAAAAAAAAAAAAAAAAAAAAAAAAAAAAAAAAAAAAGCATATGACTAAAAAAAA AAAAAAAAAAAAAAAAAAAAAAAAAAAAAAAAAAAAAAAAAAAAAAAAAAAAAAA |
| 50 | A15C65G55 | AAAAAAAAAAAAAAACAAAAAAAAAAAAAAAAAAAAAAAAAAAAAAAAAAAAAAAAAAAAAAAA AAAAAAAAAAAAAAAAGAAAAAAAAAAAAAAAAAAAAAAAAAAAAAAAAAAAAAAAAAAAAAA AAAAAAA |
| 51 | A40L80 | AAAAAAAAAAAAAAAAAAAAAAAAAAAAAAAAAAAAAAAAAAAAGCATATGACTAAAAAAAAAAAAAAAA AAAAAAAAAAAAAAAAAAAAAAAAAAAAAAAAAAAAAAAAAAAAAAAAAAAAAAAAAAAAAAAAAAAAAA |
| 52 | A50L70 | AAAAAAAAAAAAAAAAAAAAAAAAAAAAAAAAAAAAAAAAAAAAAGCATATGACTAAAAA AAAAAAAAAAAAAAAAAAAAAAAAAAAAAAAAAAAAAAAAAAAAAAAAAAAAAAAAAAAA |
| 77 | A30G69 | AAAAAAAAAAAAAAAAAAAAAAAAAAAAAGAAAAAAAAAAAAAAAAAAAAAAAAAAAAAAAA AAAAAAAAAAAAAAAAAAAAAAAAAAAAAAAAA |
| 78 | A30G70 | AAAAAAAAAAAAAAAAAAAAAAAAAAAAAAGAAAAAAAAAAAAAAAAAAAAAAAAAAAAAAAAA AAAAAAAAAAAAAAAAAAAAAAAAAAAAAAAAAAAA |
| 79 | A30G71 | AAAAAAAAAAAAAAAAAAAAAAAAAAAAAAGAAAAAAAAAAAAAAAAAAAAAAAAAAAAAAAAAA AAAAAAAAAAAAAAAAAAAAAAAAAAAAAAAAAAAAA |
| 80 | A29G70 | AAAAAAAAAAAAAAAAAAAAAAAAAAAAGAAAAAAAAAAAAAAAAAAAAAAAAAAAAAAAAAAA AAAAAAAAAAAAAAAAAAAAAAAAAAAAAAAAAA |
| 81 | A31G70 | AAAAAAAAAAAAAAAAAAAAAAAAAAAAAAAGAAAAAAAAAAAAAAAAAAAAAAAAAAAAAAAA AAAAAAAAAAAAAAAAAAAAAAAAAAAAAAAAAAAAA |

In the present disclosure, the multi-segmented PolyA polynucleotides need to be constructed onto a universal vector, which is then transformed and identified. The present disclosure has no special limitations on the universal vector, and the universal plasmid pUC-WG-kana with kana resistance is preferred. In the present disclosure, the plasmid map of pUC-GW-kana is as shown in FIG. 2. The multi-segmented PolyA polynucleotides need to be constructed at the EcoRVI insertion site on the universal vector. A synthetic plasmid for storage and transport is generally as dry powder, which is preferably to be dissolved with ultrapure water when in use. The concentration of the plasmid after water dissolution is preferably 10 ng/µl, more preferably 1 ng/µl. The present disclosure has no special limitations on the ultrapure water, and conventional laboratory ultrapure water can be used.

In the present disclosure, after obtaining the synthetic plasmid, the synthetic plasmid is transformed into different host cells to obtain recombinant cells, and colony PCR is conducted respectively for the different recombinant cells to determine the stability of the corresponding sequence of the synthetic plasmid. In the present disclosure, the different host cells are preferably Escherichia coli competent cells (including but not limited to DH5α, stbl2, stbl3, stable). The present disclosure has no special limitations on the transformation method, and transformation methods which are conventional in the art can be used. In the present disclosure, the identification of stable sequences of positive recombinant cells is preferably conducted after obtaining the recombinant cells. In the present disclosure, the screening of the positive recombinant cells is preferably carried out on a kana-containing solid medium. In the present disclosure, single colonies on the kana-containing solid medium are picked to a volume of a medium for fully dissolution to obtain a dilution of the single colonies. The present disclosure has no special limitations on the method for dissolving single colonies, and operations of picking and inoculating bacteria which are conventional in the art can be used. After obtaining the colony dilution, a small amount of the bacterial suspension is aspirated for colony PCR, and when the number of bands which are consistent with the band of interest exceeds 75%, the bacterial suspension whose amplified band is consistent with the band of interest is selected and inoculated into 100 ml of a kana-containing liquid medium for further subculture. In the present disclosure, the sequence of primer F for the colony PCR is: cgacggccagt gaattgac (SEQ ID No. 65); the sequence of primer R for the colony PCR is: cagctatgaccatgctcgag (SEQ ID No. 66). In the present disclosure, the amplification procedure of the colony PCR is preferably as follows: pre-denaturing at 98°C for 10 min; denaturing at 98°C for 300 s, annealing at 66.5°C for 15 s, and extension at 72°C for 30 s, 30 cycles; and finally extension at 72°C for 5 min. In the present disclosure, the system of the colony PCR is preferably as follows: 10 µl of 2×Hieff^{®} HotStart PCR Genotyping Master Mix (With Dye); 0.5 µl of primer F; 0.5 µl of primer R; 2 µl of the bacterial suspension; and 7 µl of water.

In the present disclosure, the initial concentration of the primer F and the primer R is preferably 10 µmol/L. In the present disclosure, the 2×Hieff^{®} HotStart PCR Genotyping Master Mix (With Dye) comprises the following components: DNA polymerase, dNTPs, Mg²⁺and nucleic acid dyes. In the present disclosure, after the PCR amplification is finished, single colonies which are consistent with the size of the band of interest are identified and analyzed preferably by agarose gel electrophoresis. The present disclosure has no special limitations on the agarose gel electrophoresis, and gel formulation and electrophoresis methods which are conventional in the art can be used. It is preferred to run on 3% agarose gel at 130V for 30 min. When the proportion of bands that are consistent with the size of the band of interest exceeds 50%, any strain whose bands are consistent with the size of the band of interest is selected and inoculated to 100 ml of a kana-containing liquid medium for strain culture. The present disclosure has no special limitations on the kana-containing liquid medium, and a kana-containing liquid medium which is conventional in the art can be used. The present disclosure has no special limitations on the method for inoculating and culturing strains, and a method for inoculating and culturing strains which is conventional in the art can be used. The culture is preferably carried out in a shaker at 220 rpm at 30°C, preferably for 16 hours. After the culture is finished, 5 µl of the bacterial suspension is subjected to 10⁴-, 10⁵-, and 10⁶-fold serial dilution respectively, and then spread onto a kana-containing solid medium and cultured overnight in an incubator at 30°C. The present disclosure has no special limitations on the dilution of the bacterial suspension, and the serial dilution method for a bacterial suspension which is conventional in the art can be used. After overnight culture, a plate with between 100 and 300 single colonies is selected for the next colony PCR identification. The steps of the identification method are the same as described above, and are respectively picking bacteria, diluting the bacterial suspension, formulating the PCR system, conducting the reaction according to specific procedures and identifying by agarose gel electrophoresis. The primers, procedures, and electrophoresis method used are the same as above. In addition, the monoclonal colonies from an appropriate plate are picked and cultured in 3 ml of a Kana-containing medium, then the plasmid miniprep and sequencing are performed, and the alignment of sequencing results can be also used as an additional evidence for stability analysis. Based on the results of the identification, the recombination rate of the plasmid with the stable sequence during the scaled-up culture of Escherichia coli can be calculated. The calculation formula for the recombination rate is: Recombination rate = 1 - Number of bands which are consistent with the size of the band of interest/Total sample size.

In the present disclosure, after obtaining all the results of the above detection experiments, some of multi-segmented PolyA sequences with an extremely low recombination rate, i.e. extremely stable, are preferentially selected and are recombined onto a vector which can express eGFP, or the PolyA sequence is added to a sequence encoding eGFP using PCR by synthesizing the primers with the corresponding stable multi-segmented PolyA sequence; and the corresponding mRNA is prepared by *in vitro* transcription method and transfected into 293T/17 cells for a cell-level translation verification. The effect of different PolyA sequences on translation ability is analyzed by qualitative analysis with inverted fluorescence microscopy and quantification of fluorescence intensity with a flow cytometer.

The technical solutions provided by the present disclosure are described below in detail in connection with the examples, which cannot be construed as limitations on the scope of protection of the present disclosure.

### Example 1

### Design and synthesis of multi-segmented PolyA polynucleotides:

1) DNA segments of interest of SEQ ID No. 1 to SEQ ID No. 52, and SEQ ID NO. 77 to SEQ ID NO. 81 were synthesized, the design schematic diagram of the composition of the above sequences was shown in FIG. 1, and the above sequences were constructed at the EcoRV I site on the universal vector pUC-GW-kana, and the map of the universal vector was shown in FIG. 2;
2) the optimal annealing temperature for universal primers for colony PCR was identified ;
3) the gene of interest was transformed into Escherichia coli competent stbl2 or stbl3 and the bacteria were cultured in a kana-containing liquid medium;
4) preliminary identification was performed by colony PCR;
5) the bacterial suspension identified to met the requirements was inoculated, cultured, serial-diluted, and spread to perform identification and analysis;
6) sequencing analysis of the plasmid of interest in the strain was performed after scaled-up culture; and
7) stability data for all sequences was collected and analyzed.

### The steps of identifying the optimal annealing temperature were as follows:

The enzyme, primers and the plasmid template were removed from -20°C and thawed on an ice-water mixture until all the materials were thawed completely, without visible solid ice. The PCR reaction system was formulated, the specific system was as follows:

| | |
|---|---|
| 2×Hieff^{®} HotStart PCR Genotyping Master Mix (With Dye) | 200 µl |
| Primer F | 10 µl |
| Primer R | 10 µl |
| Plasmid | 10 µl |
| Water | 170 µl. |

In the present disclosure, the initial concentration of the primer F and the primer R was 10 µmol/L; the concentration of the DNA template (a plasmid with the sequence shown in SEQ ID No. 1) was 1 ng/µl. In the present disclosure, the sequence of the primer F was as follows: cgacggccagtgaattgac (SEQ ID No. 65); the sequence of the primer R was as follows: cagctatgaccatgctcgag (SEQ ID No. 66). The formulated reaction system was aliquoted into 19 different PCR tubes, 20 µl each tube. The different tubes were placed in sequence on a uniform horizontal row in a PCR instrument, and the gradient annealing PCR program was set for investigation and detection of the optimal annealing temperature.

In the present disclosure, the amplification procedure of the above gradient annealing PCR was preferably as follows: pre-denaturing at 98°C for 10 min; denaturing at 98°C for 30 s, annealing at 55.7 to 66.5°C for 15 s, and extension at 72°C for 30 s, 30 cycles; and finally extension at 72°C for 5 min. This procedure allowed for each well in the horizontal row in the PCR instrument to correspond to different annealing temperatures, while the other temperature change procedures were identical, which can obtain in a short time the difference of products from the same system with only the annealing temperature changed, thereby analyzing and obtaining the optimal annealing temperature for the primers.

After the above procedure was finished, a 3% agarose gel was formulated (weighing 3 g of agarose into 100 ml of TAE solution), 0.5 µl of bromophenol blue was added to the PCR tube in which the PCR reaction had been completed, and the mixture was mixed well by shaking, and then the samples were loaded in different temperature order, the electrophoresis program was set at 130V and ran 30 min followed by color development, and the detection results are as shown in FIG. 3. In order to reduce the effect of non-specific bands on the detection results, the annealing temperature of 66.5°C at which non-specific bands were minimal in number was selected for subsequent experiments.

### The steps of plasmid transformation were as follows:

A corresponding number of clean 1.5 ml EP tubes were taken on the EP tube rack, and the label information corresponding to the plasmid to be transformed was written; tubes of dry powder of synthetic genes (synthetic gene plasmids comprising one of the sequences SEQ ID No. 1 to SEQ ID No. 52, and SEQ ID NO. 77 to SEQ ID NO. 81, respectively) were taken and centrifuged at 10000G for 1 min in a high speed centrifuge, diluted to 10 ng/µl with enzyme-free water in an operation area, and then inserted into ice for pre-cooling for 5 min; meanwhile, the competent stbl2 and stbl3 were taken and thawed on ice, and aliquoted in 1.5 ml prepared EP tubes after the competent cells had no visible ice flakes, 50 µl each tube; and the pre-cooled synthetic plasmids corresponding to the labels at 1 ng per tube were added to the competent cells. The tubes were incubated on ice for 30 min after mixing well by gently pipetting with a pipette tip, and then the EP tubes were placed in a pre-heated 42°C water bath for heat shock, which needed to immerse the bacteria under the liquid surface for fully heating. After heat shock for 45 s, the tubes were removed and gently inserted under the ice surface. The tubes were incubated for 2 min, to which 950 µl of LB liquid medium was added, and the mixture was resuscitated in a shaker at 220 rpm at 30°C for 1 h. 50 µl, 100 µl, and 150 µl of bacteria were respectively spread on a kana-containing solid medium in a plate. After incubating in an incubator at 30°C for 20 to 24 h, a plate with single colonies which were visible and approximately 100 to 300 in number was selected for identification.

**The steps of colony PCR were as follows:**
Single colonies picking: The corresponding number of eight-tube strips were placed into a PCR tube rack, LB medium was poured into a reservoir for a multi-channel pipette, and then 50 µl of LB medium was added into the eight-tube strips with the multi-channel pipette; single colonies on the plate were picked with a sterilized small and white pipette tip hold with forceps and placed in the eight-tube strips, the PCR tube rack was gently shaken such that the bacteria on the pipette tip were mixed well in the medium; the small and white pipette tip was removed, and the eight-tube strips were capped and the caps were marked with an anti-alcohol marker; and the eight-tube strips were incubated in a shaker at 30°C for 2 h and the relevant records (date, numbering, etc.) were written on a log book. After removing from the shaker, the eight-tube strips were spun briefly in a pocket centrifuge and bubbles were flicked away gently with fingers, and then the eight-tube strips were spun briefly for another 15 s and stored at 4°C for later use.

Colony PCR reaction: The following PCR reaction was formulated, the formulated reaction mixture was added to a 96-well plate and 1 µl of the bacterial suspension was added thereto using a multi-channel pipette; an additional reaction with the original gene plasmid as a template needed to be set as a positive control, and a reaction with water as a template served as a negative control. Amplification was performed according to the PCR procedure:

| | |
|---|---|
| 2×Hieff^{®} HotStart PCR Genotyping Master Mix (With Dye) | 10 µl |
| Primer F | 0.5 µl |
| Primer R | 0.5 µl |
| Bacterial suspension | 2 µl |
| Water | 7 µl. |

In the present disclosure, the initial concentration of the primer F and the primer R was 10 µmol/L; the concentration of the DNA template was 1 ng/µl. In the present disclosure, the sequence of the primer F was as follows: cgacggccagtgaattgac (SEQ ID No. 65); the sequence of the primer R was as follows: cagctatgaccatgctcgag (SEQ ID No. 66). In the present disclosure, the amplification procedure of the PCR was preferably as follows: pre-denaturing at 98°C for 10 min; denaturing at 98°C for 30 s, annealing at 66.5°C for 15 s, and extension at 72°C for 30 s, 30 cycles; and finally extension at 72°C for 5 min.

Agarose gel electrophoresis: 3% agarose gel was firstly formulated (weighing 3 g of agarose into 100 ml of TAE solution); 0.5 µl of bromophenol blue was added to the eight-tube strips in which the PCR reaction had been completed, and the mixture was mixed well by shaking and then the samples were loaded; and the picture of electrophoresed agarose gel was taken and saved.

Definition and analysis of the recombination rate: the recombination rate of a stable sequence was determined by the agarose gel electrophoresis band diagram, and if the colony PCR band was comparable in size to that of the positive control (the PCR template was the corresponding plasmid) and had no impurity band, the bacteria were counted as non-recombinant strains, otherwise they were counted as recombinant strains. Recombination rate = Number of recombinant strains counted/Total number of test strains.

Post-scaled-up-culture stability detection of preferred sequence plasmids after recombination rate analysis: based on the above screening, some of sequences with the recombination rate in preliminary screening of less than 15% (including SEQ ID No. 44, SEQ ID No. 45, SEQ ID No. 46, SEQ ID No. 47, and SEQ ID No. 51) were screened for further scaled-up culture and detection. Stable strains identified by the colony PCR were selected, 10 µl of the bacterial suspension was aspirated and inoculated into 100 ml of a kana-containing LB liquid medium and cultured at 30°C/220 rpm for 16 h. The bacterial suspension was subjected to 10⁴-, 10⁵-, and 10⁶-fold dilution respectively, and then spread onto a kana-containing solid medium. After culturing in an incubator at 30°C for 20 h, the plate with more homogenous colonies was selected, several single colonies were picked for stability detection, and the detection method was the same as above.

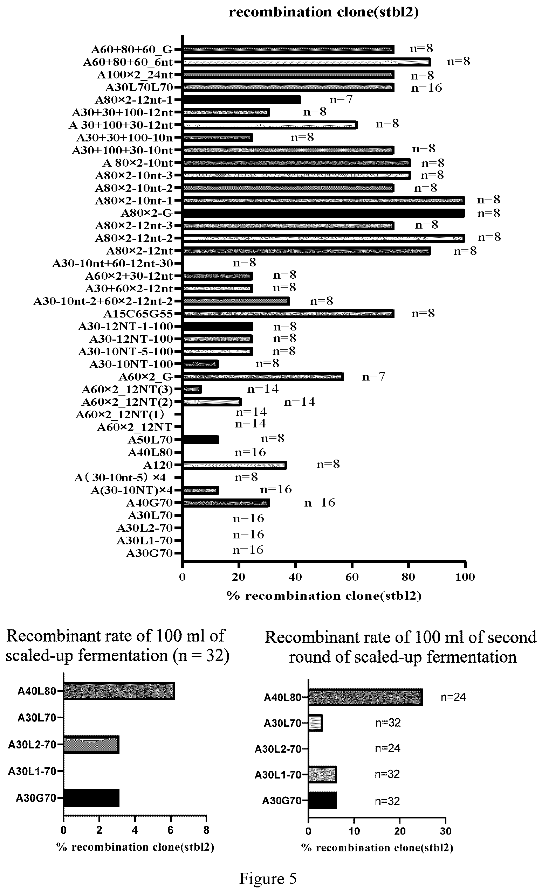

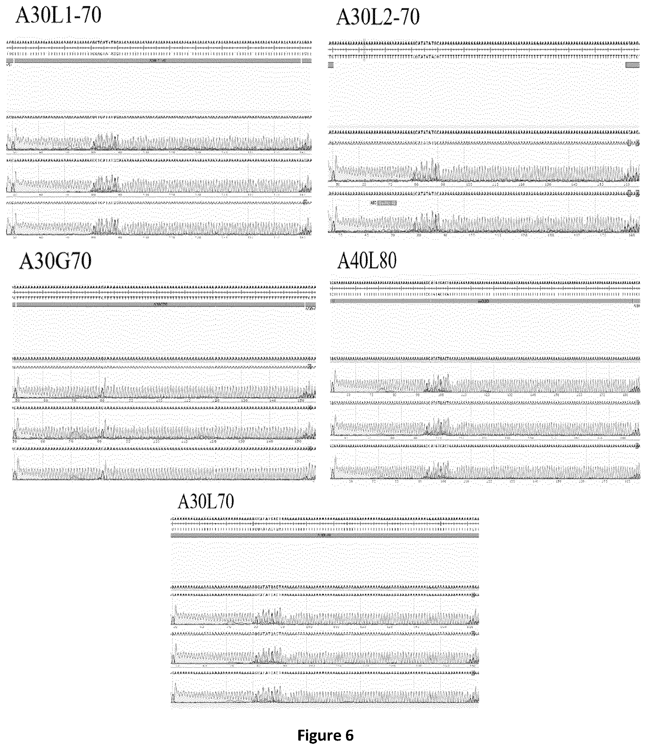

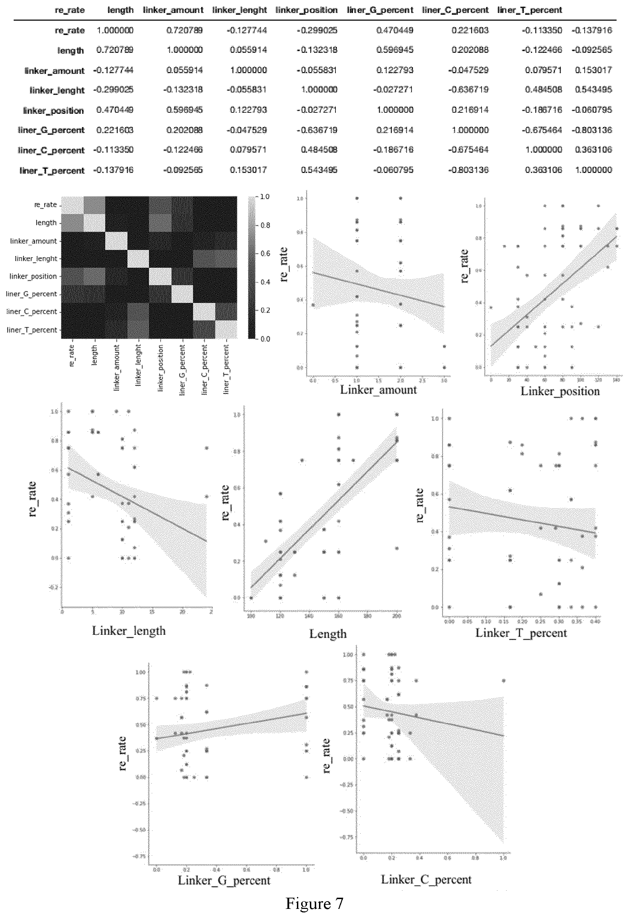

The detection results of the recombination rate after the transformation of some of plasmids into the stbl3 strain are as shown in FIG. 4, and the detection results after the transformation of some of plasmids into the stbl2 strain and the detection results after the scaled-up fermentation of stable strains are as shown in FIG. 5. For more accurate determination of the stability of sequences, the strain after scaled-up culture was entrusted to an external institution for sequencing, and the results of sequencing and alignment are as shown in FIG. 6. Based on the numerous detection results in FIGS. 4 and 5, in order to more intuitively analyze the relationship between the stability of multi-segmented PolyA and features of sequences, data was processed using matplotlib.pyplot, and correlation analysis between each of sequence features (which were disassembled into linker amount, position, length and the contents of G, C, and T) and the stability of the multi-segmented PolyA polynucleotides were performed, and the results are as shown in FIG. 7.

### Example 2

### Translation validation of some of multi-segmented PolyA sequences:

1) a linearized vector backbone and a multi-segmented PolyA sequence with ends homologous to the backbone were obtained by the PCR method;
2) the linearized backbone was connected to the PolyA sequence by homologous recombination;
3) the connection product was transformed into Escherichia coli stbl2, the bacterial suspension was spread on a kana-containing LB solid screening medium and cultured overnight in an incubator at 30°C to achieve a preliminary recombinant screening;
4) final correct recombinants were confirmed by sequencing;
5) corresponding transcription templates were obtained by linearization of recombinants and PCR tailing;
6) corresponding mRNAs were prepared by *in vitro* transcription; and
7) cell translation validation was performed.

**The PCR steps for obtaining the linearized backbone and PolyA sequences were** as **follows:**
PCR reaction: a kana resistant plasmid with T7 promoter, 5' UTR, an eGFP coding region and 3' UTR sequence was obtained by gene synthesis and used as a backbone for the construction of the above vector of interest. Linearization was performed by the PCR method, and the PCR reaction system comprised: 10 µl of PrimeSTAR^{®} Max DNA Polymerase; 0.5 µl of primer F; 0.5 µl of primer R; 0.5 µl of the plasmid template; and 8.5 µl of water.

In the present disclosure, the initial concentration of the primer F and the primer R was 10 µmol/L; the concentration of the DNA template was 1 ng/µl. In the present disclosure, the sequence of the primer F for the linearized vector backbone was as follows: TGAGGGTCTAGAACTAGTGTCG (SEQ ID No. 67); the sequence of the primer R was as follows: CTTCCTACTCAGGCTTTATTCAAAG (SEQ ID No. 68); and the plasmid template used was a synthetic gene with the above backbone sequence.

The PCR system used to amplify PolyA segments with sequences homologous to the backbone was the same as above, wherein the sequence of the primer F used was as follows: CTTTGAATAAAGCCTGAGTAGGAAG (SEQ ID No. 69); the sequence of the primer R used was as follows: CGACACTAGTTCTAGACCCTCA (SEQ ID No. 70); and the plasmid template used was a synthetic gene with a multi-segmented stable PolyA sequence, wherein both ends of the gene comprised sequences homologous to the backbone, the 5' end flanking sequence was: CTTTGAATAAAGCCTGAGTAGGAAG (SEQ ID No. 71), and the 3' end flanking sequence was: CGACACTAGTTCTAGACCCTCA (SEQ ID No. 72). The selected multi-segmented PolyA sequences for construction included A30L70, A30L₁70, A30L₂70, A30G70, and A40L80.

In the present disclosure, the amplification procedure of the above PCR was preferably as follows: pre-denaturing at 98°C for 10 min; denaturing at 98°C for 30 s, annealing at 55°C for 15 s, and extension at 72°C for 30 s, 30 cycles; and finally extension at 72°C for 5 min.

Agarose gel electrophoresis: 1% agarose gel was firstly formulated (weighing 1 g of agarose into 100 ml of TAE solution); 0.5 µl of bromophenol blue was added to the PCR tubes in which the PCR reaction had been completed, and the mixture was mixed well by shaking and then the samples were loaded, and a DNA marker was loaded on the lane near the samples; and the picture of electrophoresed agarose gel was taken and saved.

Analysis of a band of interest: The gel was developed, and the band of interest was compared with the corresponding-sized band of the marker to confirm that the band of interest was clear and of the correct size. The above steps were repeated if the band size was incorrect or no band was detected.

The steps of *in vitro* recombination for the band of interest were as follows:
Formulation of a recombination system: The in vitro recombination system was formulated using the above PCR products: 2 µl of a PCR product of the backbone, 2 µl of a PCR product of PolyA, 0.1 µl of T5 Exonuclease, 0.5 µl of 4 buffer, and 0.4 µl of ddH₂O. The entire formulation of the system was completed on ice. After the formulation was completed, the system was mixed well by a pipette gently and incubated on ice for 5 min. After the incubation was completed, 50 µl of Escherichia coli competent cells, preferably stbl2 competent cells were added into the system, and the system was pipetted gently and continued to be incubated on ice for 30 min. Then the system was placed into a 42°C water bath for heat shock for 45 s and then put back on ice and incubated for 2 min. The system was removed, to which 950 µl of LB liquid medium was added, and the mixture was resuscitated in a shaker at 30°C for 1 h, spread on a kana-containing LB solid medium and cultured in an incubator at 30°C for 20 h.

Screening of final correct recombinants: After culturing the above spread medium for 20 h, and several single colonies were picked, inoculated to 3ml of a kana-containing LB liquid medium and cultured at 30°C for 16 h. Plasmids were extracted and sequenced, the sequencing results were aligned with the designed sequences, and the plasmids with correct alignment results were numbered and aliquoted for storage.

**The steps for preparing the transcription template by the plasmid method were as follows:**
Linearization of recombinant plasmids: The constructed recombinant plasmid was linearized using an enzymatic cleavage site of a designed type IIS restriction enzyme, and in the present disclosure, BspQI was preferably used, and the enzymatic cleavage system was as follows: 100 µg of the recombinant plasmid (1 µg/µl), 500 µl of NEBuffer^{™} r3.1, 30 µl of BspQI (10000 unit/ml) and 4.37 ml of sterile water. The above materials were mixed well and incubated in a metal bath at 50°C for 1 h. After the incubation is finished, purification was performed using the Cycle Pure Kit, referring to the kit instructions for the operating method.

Detection of linearized products: 2 µl of the above linearized product purified was taken for concentration determination using nanodrop microvolume spectrophotometer, the concentration of the product was recorded, and 400 ng of the product was mixed with 10× DNA loading buffer for agarose gel electrophoresis detection. Bands were single, clear and of correct size, and labeled for storage at -20°C or for the next step of *in vitro* transcription.

**The steps of the PCR method for preparing the transcription template were as follows:**
Formulation of PCR system: A kana resistant plasmid with T7 promoter, 5' UTR, an eGFP coding region and 3' UTR sequence was obtained by gene synthesis, and a PolyA tail was added to the above continuous sequence by the PCR method, wherein the PCR reaction system was:

| | |
|---|---|
| PrimeSTAR^{®} Max DNA Polymerase | 200 µl |
| Primer F | 4 µl |
| Primer R | 4 µl |
| Plasmid template | 4 µl |
| Water | 188 µl |

In the present disclosure, the initial concentration of the primer F and the primer R was 10 µmol/L; the concentration of the DNA template was 1 ng/µl. In the present disclosure, the sequence of the primer F was as follows: GTACAGAAGCTAATACGACTCAC (SEQ ID No. 73); the sequence of the primer R was as follows: TTTTTTTTTTTTTTTTTTTTTTTTTTTTTT TTTTTTTTTTTTTTTTTTTTTTTTTTTTTTTTTTTTTTTTTTTTTTTTTTTTTTTTTTTTTTTTTTTTTTTTTTTTTTTT TTTTTTTTTTCTTCCTACTCAGGCTTTATTCAAAG (SEQ ID No. 74) or TTTTTTTTTTTTTTTTTTTTTTTTTTTTTTTTTTTTTTTTTTTTTTTTTTTTTTTTTTTTTT TTTTTTTTCTTTTTTTTTTTTTTTTTTTTTTTTTTTTTTCTTCCTACTCAGGCTTTATTCAA AG (SEQ ID No. 75) or TTTTTTTTTTTTTTTTTTTTTTTTTTTTTTTTTTTTTTTTTTTTTTT TTTTTTTTTTTTTTTTTTTTTTTTTTTTTTTTTAGTCATATGCTTTTTTTTTTTTTTTTTTTTTTTTTTTTTTTTTTTT TTTTCTTCCTACTCAGGCTTTATT (SEQ ID No. 76); the plasmid template used was the above synthetic gene with T7 promoter, 5' UTR, the eGFP coding region and 3' UTR sequence.

In the present disclosure, the amplification procedure of the above PCR was preferably as follows: pre-denaturing at 98°C for 10 min; denaturing at 98°C for 30 s, annealing at 55°C for 15 s, and extension at 72°C for 30 s, 30 cycles; and finally extension at 72°C for 5 min.

Purification of PCR products: 0.5 g of agarose was weighed into 50 ml of 1×TAE, the mixture was heated in a microwave oven until no obvious solids were visible; 5 µl of a nucleic acid dye was added, the mixture was mixed well and poured into a pre-prepared gel-casting device to prepare recovery gel with 400-µl wells. The gel was left standing at room temperature for 30 min until the gel was completely coagulated, then the comb was pulled out, and the gel was placed into an electrophoresis apparatus. All the above PCR product mixed with DNA loading buffer was loaded in the electrophoresis apparatus for running at 130 V for 30 min, and then the gel was transferred to a blue light gel imager for analysis of the band of interest. After confirming that the band of interest was clear and of correct size, the band of interest was cut off with a knife sterilized with alcohol and placed in an EP tube. Subsequent purification was performed using the E.Z.N.A.^{®}Gel Extraction Kit, referring to the kit instructions for specific operations.

Detection of purified products: 2 µl of the above PCR product purified was taken for concentration determination using nanodrop microvolume spectrophotometer, the concentration of the product was recorded, and 400 ng of the product was mixed with 10× DNA loading buffer for agarose gel electrophoresis detection. Bands were single, clear and of correct size, and labeled for storage at -20°C or for the next step of *in vitro* transcription.

### Preparation of mRNA by in vitro transcription:

Formulation of transcription systems: The different templates were respectively used to formulate *in vitro* transcription systems for the preparation of corresponding mRNAs, and the specific reaction system was as follows:

| | |
|---|---|
| NTP | 2 µl |
| CAP | 2 µl |
| 10xbuffer | 2 µl |
| T7 RNA polymerase mix | 2 µl |
| Transcription template | 400 ng |
| Water | added to make up to 20 µl |

The above system formulated was placed in a PCR instrument, and the procedure was set to react at 37°C for 4 h; 2 µl of DNAse I was added to the reaction system, and the reaction was continued in an environment at 37°C for 45 min; the reaction system was removed and purified using the MEGAclean^{™} Kit, and for specific purification steps, the kit instructions can be referred to.

Detection of mRNAs: 2 µl of the above mRNAs purified were taken for concentration determination using nanodrop microvolume spectrophotometer, the concentration of the product was recorded, and 400 ng of the product was mixed with 10× RNA loading buffer for agarose gel electrophoresis detection. Bands were single, clear and of correct size, and labeled for storage at -20°C or for the next step of detection experiments.

### Cell translation verification of mRNAs with different multi-segmented PolyA sequences for expressing GFP:

### Preparation of cells and transfection reagents:

Host cells used for transfection were 293T, and the cells were inoculated into a 24-well plate in advance such that they could reach 70% to 90% confluence at transfection. The medium was exchanged with opti-MEMTM medium containing 5% serum at 0.5 to 4 h prior to transfection. Subsequent transfection needed to be performed with the transfection kit Lipofectamine^{™} 3000, and the specific steps were as follows:
The mRNAs and the transfection reagent Lipofectamine^{™} 3000 were diluted in a proportion using opti-MEMTM medium at the same time, and then the diluted Lipofectamine^{™} 3000 was mixed with the diluted mRNAs in a 1 : 1 ratio, wherein the formulation systems of the dilution were as follows: 1) 25 µl of opti-MEMTM medium, and 1.5 µl of Lipofectamine^{™} 3000; 2) 50 µl of opti-MEMTM medium, and 1 µg of the corresponding mRNAs. After fifteen minutes of incubation, the mixture was added dropwise into a pre-prepared well plate containing the host cells, and incubated in a carbon dioxide incubator for 6 to 24 h; the medium was exchanged with a complete medium and corresponding detection was carried out.

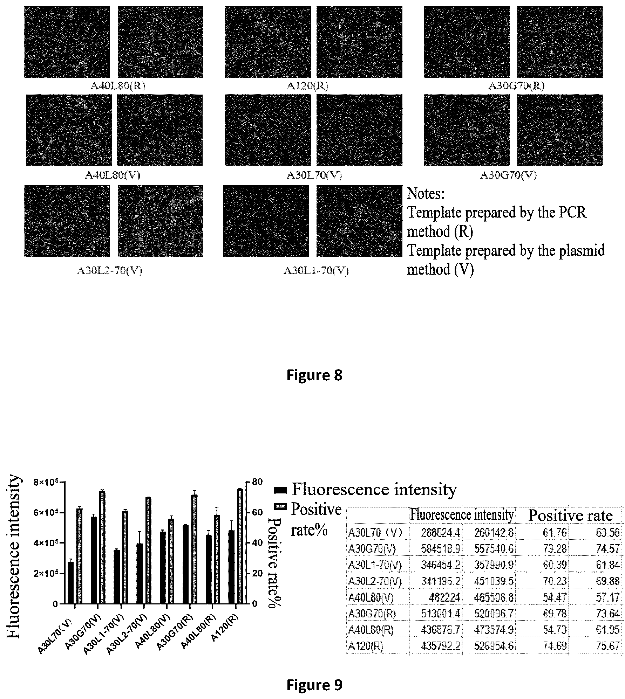

Inverted fluorescence microscopy: After 24 h of culture, the cell culture plate was removed from the carbon dioxide incubator and placed under the lens of an inverted fluorescence microscope; the corresponding channels and detection parameters were set, and firstly the fluorescence in the field of view was observed using a low power objective lens; when the lens stayed in a field of view where the cells were evenly distributed and showed strong fluorescence, the lens can be switched to a high power objective lens for observation and photographing; pictures were saved and named for storage and recording. The detection results are as shown in FIG. 8.

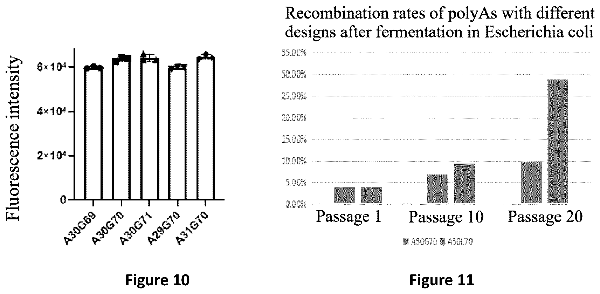

Flow cytometry detection: The cells that were correct under the above microscopy were digested with trypsin at room temperature for 1 min, and the digestion reaction was terminated with a complete medium; the mixture were pipetted into a 1.5 ml sharp-bottom EP tube by a pipette for centrifugation at 1000 g, the supernatant was discarded and the resting was washed twice with PBS; the sample detection was performed with a flow cytometer, the mean fluorescence intensity was detected with the FITC channel, and the blank cell (which was not transfected with the mRNAs) was used as the negative control. The fluorescence intensity expressed by cells with different mRNAs was analyzed by comparing the detection results, and the detection results are as shown in FIG. 9. The detection results of the expression level of mRNAs corresponding to the multi-segmented PolyAs as shown in SEQ ID No. 77 to SEQ ID No. 81 are as shown in FIG. 10.

As can be seen from the example described above, the multi-segmented PolyA polynucleotides provided by the present disclosure can stably exist during the passage of Escherichia coli.

### Stability comparison of A30G70 and A30L70 after extreme scaled-up culture

### (1) Transformation of plasmids containing the gene of interest

Plasmids with A30G70 and A30L70 were re-transformed into Escherichia coli competent cells, and the specific operation steps were as follows: the synthetic gene was diluted to 1 ng/µl with enzyme-free water; Escherichia coli competent cells stbl2 were removed from a -80°C freezer and placed on an ice-water mixture to stand still, and after 5 min, the competent cells were completely thawed; sterile 1.5 ml EP tubes were taken and the corresponding labels were written on them, 50 µl of the competent cells were added to each tube, and then 1 µl of the plasmids were added successively according to the labels on the tubes, and the competent cells and the plasmids were mixed by gently pipetting 5 times. It should be ensured that the operation process can not leave the ice-water mixture to ensure a low-temperature environment, and the operation should be as gentle as possible to reduce the effect on the competence efficiency. The competent cells were mixed well with the plasmids and placed on an ice-water mixture for incubation; after 30 min, a heat-shock was performed, i.e. the mixture was incubated in a 42°C water bath for 45 s; hereafter, the mixture was removed immediately from the water bath and placed again on the ice-water mixture for standing for 2 min; 950 µl of LB liquid medium was added into each tube in a sterile operation area, and the EP tubes were incubated in a shaking incubator at 30°C for 1 h; the tubes were removed from the shaking incubator at the end of the incubation, and 100 µl of the mixture was pipetted from each tube with a pipette and spread on a kana-containing solid medium in a plate for the kana resistance screening. After spreading evenly, the plate was inverted in an incubator at 30°C for static cultivation for 20 h. Acceptable criteria were that single colonies were visible on the plate, approximately 100 to 300 in number, which could be used for the next step of identification.

### (2) Fermentation stability validation

The corresponding number of eight-tube strips were placed into a PCR tube rack, LB medium was poured into a reservoir for a multi-channel pipette, and then 120 µl of LB medium was added into the eight-tube strips with the multi-channel pipette; single colonies on the plate were picked with a sterilized small and white pipette tip hold with forceps and placed in the eight-tube strips, the PCR tube rack was gently shaken such that the bacteria on the pipette tip were mixed well in the medium; the small and white pipette tip was removed, and the eight-tube strips were capped and the caps were marked with an anti-alcohol marker; and the eight-tube strips were incubated in a shaker at 30°C for 2 h and the relevant records (date, numbering, etc.) were written on a log book. After removing from the shaker, the eight-tube strips were spun briefly in a pocket centrifuge and bubbles were flicked away gently with fingers, and then the eight-tube strips were spun briefly for another 15 s and stored at 4°C for later use.

Colony PCR reaction: The following PCR reaction was formulated, the formulated reaction mixture was added to a 96-well plate and 1 µl of the bacterial suspension was added thereto using a multi-channel pipette; an additional reaction with the original gene plasmid as a template needed to be set as a positive control, and a reaction with water as a template served as a negative control. Amplification was performed according to the PCR procedure:

| | |
|---|---|
| 2×Hieff^{®} HotStart PCR Genotyping Master Mix (With Dye) | 10 µl |
| Primer F | 0.5 µl |
| Primer R | 0.5 µl |
| Bacterial suspension | 2 µl |
| Water | 7 µl. |

In the present disclosure, the initial concentration of the primer F and the primer R was 10 µmol/L; the concentration of the DNA template was 1 ng/µl. In the present disclosure, the sequence of the primer F was as follows: 5'-gtaaaacgacggccagt-3' (SEQ ID No. 82); the sequence of the primer R was as follows: 5'-caggaaacagctatgac-3' (SEQ ID No. 83). In the present disclosure, the amplification procedure of the PCR was preferably as follows: pre-denaturing at 98°C for 10 min; denaturing at 98°C for 30 s, annealing at 60°C for 15 s, and extension at 72°C for 30 s, 30 cycles; and finally extension at 72°C for 5 min.

Agarose gel electrophoresis: 3% agarose gel was firstly formulated (weighing 3 g of agarose into 100 ml of TAE solution); 0.5 µl of bromophenol blue was added to the eight-tube strips in which the PCR reaction had been completed, and the mixture was mixed well by shaking and then the samples were loaded; and the picture of electrophoresed agarose gel was taken and saved. The recombination rate of a stable sequence was determined by the agarose gel electrophoresis band diagram, and if the colony PCR band was comparable in size to that of the positive control and had no impurity band, the bacteria were defined as non-recombinant strains, otherwise they were determined as recombinant strains. A non-recombinant strain was selected for scaled-up culture at a volume ratio of 1 : 1000 at 30°C for 24 h, which was defined as one generation of culture; after the culture was finished, 100 µl of the bacterial suspension was pipetted and inoculated into 100 ml of a fresh medium for continuous culture. The culture was continued for 20 passages, the stability of the intermediate and final passages was detected, and the detection method was the same as above.

The detection results are as shown in FIG. 11, and it can be analyzed that A30G70 shows a significant advantage after extreme scaled-up culture. After 20 passages of extreme scaled-up fermentation, the recombination rate of A30G70 is only less than 10, almost one third of that of A30L70, predicting that after the process has been scaled up to a certain extent, A30G70 will bring the quality of mRNA products to a higher level.

The foregoing contents are only preferred embodiments of the present disclosure, and it should be noted that for one of ordinary skill in the technical field, without departing from the principles of the present disclosure, several improvements and modifications may also be made, and the improvements and modifications should also be considered within the scope of protection of the present disclosure.

## Claims

1. A multi-segmented PolyA polynucleotide comprising two or more PolyA segments, wherein adjacent PolyA segments are connected via a linker, each PolyA segment is composed of a plurality of continuous As, and each linker is composed of 1 to 24 nt nucleotide residues that are not all As.

2. The multi-segmented PolyA polynucleotide according to claim 1, which is selected from one or more of polynucleotides having the following structures:
PolyA segment 1-linker 1-PolyA segment 2;
PolyA segment 1-linker 1-PolyA segment 2-linker 2-PolyA segment 3; or
PolyA segment 1-linker 1-PolyA segment 2-linker 2-PolyA segment 3-linker 3-PolyA segment 4.

3. The multi-segmented PolyA polynucleotide according to claim 1 or 2, wherein each PolyA segment is independently composed of 10 to 100 continuous As;
preferably, the PolyA segment 1 is composed of 30 to 60 continuous As;
preferably, each PolyA segment is independently composed of 30, 40, 50, 60, 70, 80, 90 or 100 continuous As;
preferably, the total number of As in all the PolyA segments in the multi-segmented PolyA polynucleotide is 30 to 300, preferably 60 to 240, more preferably 100 to 200.

4. The multi-segmented PolyA polynucleotide according to claim 1, wherein each linker is composed of 1 to 12 nt nucleotide residues that are not all As;
preferably, each linker is composed of G, or has a G content of 20% to 33% among all bases in the linker sequence.

5. The multi-segmented PolyA polynucleotide according to any one of claims 1 to 3, wherein each linker is independently selected from the nucleotides represented by the following sequences:
G;
C;
GGGGGG;
ATGCAT;
GCATATGACT;
CAGTAATGAC;
AGTCATATGC;
GTCATTACTG;
TGTCAGATAC;
GCTCATATGC;
GCATATATGC;
CGCCATTAGAGG;
ATGCATGATATC;
TGCAACATCGAT;
CCTCTAATGGCG; and
CAACCCCTGATTGTGTCCGCATCT;
preferably, in each multi-segmented PolyA polynucleotide, the sum of the lengths of all linkers is 1 to 48 nt, more preferably 2 to 24 nt.

6. The multi-segmented PolyA polynucleotide according to any one of claims 1 to 5, comprising one or more of polynucleotides represented by SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9, SEQ ID NO. 10, SEQ ID NO. 11, SEQ ID NO. 12, SEQ ID NO. 13, SEQ ID NO. 14, SEQ ID NO. 15, SEQ ID NO. 16, SEQ ID NO. 17, SEQ ID NO. 18, SEQ ID NO. 19, SEQ ID NO. 20, SEQ ID NO. 21, SEQ ID NO. 22, SEQ ID NO. 23, SEQ ID NO. 24, SEQ ID NO. 25, SEQ ID NO. 26, SEQ ID NO. 27, SEQ ID NO. 28, SEQ ID NO. 29, SEQ ID NO. 30, SEQ ID NO. 31, SEQ ID NO. 32, SEQ ID NO. 33, SEQ ID NO. 34, SEQ ID NO. 35, SEQ ID NO. 36, SEQ ID NO. 37, SEQ ID NO. 38, SEQ ID NO. 39, SEQ ID NO. 40, SEQ ID NO. 41, SEQ ID NO. 42, SEQ ID NO. 43, SEQ ID NO. 44, SEQ ID NO. 45, SEQ ID NO. 46, SEQ ID NO. 47, SEQ ID NO. 48, SEQ ID NO. 49, SEQ ID NO. 50, SEQ ID NO. 51, SEQ ID NO. 52, SEQ ID NO. 77, SEQ ID NO. 78, SEQ ID NO. 79, SEQ ID NO. 80, and SEQ ID NO. 81.

7. A vector comprising the multi-segmented PolyA polynucleotide according to any one of claims 1 to 6.

8. A host cell comprising the multi-segmented PolyA polynucleotide according to any one of claims 1 to 6 or the vector according to claim 7.

9. Use of the multi-segmented PolyA polynucleotide according to any one of claims 1 to 6, the vector according to claim 7, or the host cell according to claim 8 in the production of an mRNA of interest through *in vitro* transcription; preferably, wherein the mRNA of interest is for use in the production of mRNA vaccines or medicaments.

10. An mRNA comprising a poly-A tail generated by *in vitro* transcription of the multi-segmented PolyA polynucleotide according to any one of claims 1 to 6.

11. A method for assessing the stability of a multi-segmented PolyA polynucleotide in a host cell, the method comprising:
1) cloning the multi-segmented PolyA polynucleotide according to any one of claims 1 to 6 into a universal plasmid vector to obtain a plasmid vector inserted with the multi-segmented PolyA polynucleotide;
2) transforming the plasmid vector inserted with the multi-segmented PolyA polynucleotide into a host cell such as *Escherichia coli* to obtain a recombinant cell, spreading the recombinant cells on a solid medium in a plate to obtain single colonies, picking the single colonies for preliminary identification by PCR, and screening for stable colonies without recombination; wherein, preferably, PCR products are identified by 2% to 3% agarose gel electrophoresis; and
3) performing scaled-up fermentation of the stable colonies without recombination screened by the preliminary identification, spreading serial-diluted cultures onto a solid medium in a plate, and carrying out PCR of colonies to assess the passage stability.
